# EUROPEAN PATENT SPECIFICATION

(11) **EP 3 964 258 B1**
(45) Date of publication and mention of the grant of the patent: **06.11.2024**
(21) Application number: 20194501.1
(22) Date of filing: 04.09.2020
(51) Int. Cl.: A61N 1/04, C09J 133/00, C08K 5/34

(54) **A DRY STIMULATION ELECTRODE**
TROCKENE STIMULATIONSELEKTRODE
ÉLECTRODE DE STIMULATION SÈCHE

(43) Date of publication of application: 09.03.2022
(73) Proprietor: Henkel AG & Co. KGaA, 40589 Düsseldorf (DE)
(72) Inventor: Roschek, Tobias, 42699 Solingen (DE); Negele, Carla, 41472 Neuss (DE); Goethel, Frank, 09125 Chemnitz (DE); van der Meulen, Inge, 5521 WM Eersel (NL)

(56) References cited:
- WO-A1-2020/180186
- WO-A2-2010/033909
- US-A- 6 121 508
- US-A1- 2018 086 948
- US-A1- 2019 048 193
- MEHMET ISIK ET AL: "Cholinium-based ion gels as solid electrolytes for long-term cutaneous electrophysiology", JOURNAL OF MATERIALS CHEMISTRY C, vol. 3, no. 34, 1 January 2015 (2015-01-01), GB, pages 8942 - 8948, XP055709293, ISSN: 2050-7526, DOI: 10.1039/C5TC01888A

## Description

### Technical field of the invention

The present invention is defined in the appended claims and relates to a stimulating electrode comprising an ionically conductive pressure sensitive adhesive allowing prolongated stimulation times without skin irritation and loss of signal quality.

### Background of the invention

Various kinds of electrodes are used to provide an electrical stimulation. By the term electrical stimulation is meant herein any application where an electrical signal is transferred to the body via the skin. Examples of such applications are transcutaneous electrical nerve stimulation (TENS), electrical muscle stimulation (EMS), functional electrical stimulation (FES), wound care, electrosurgery, defibrillation, electrodermal activity (EDA) and total body electrical conductivity.

Electrical stimulation can feature significantly high currents compared to measuring bio-signals. However, high local current densities can feel unpleasant or even cause a pain and damage, therefore, higher electrode areas should be used, especially for stimulation of deeper lying muscles where higher currents are required for stimulation. Larger electrode areas are obviously more critical for current distribution because the resistance of the conductive layer will be more critical. Furthermore, high local current densities may be caused by dissimilarities in the skin resistance (e.g. hair follicles, sweat glands).

For many stimulation applications it is also beneficial to leave the electrode on the body for a prolonged duration of time (e.g. TENS and wound care). Currently used electrodes are connected to the skin via gel, which acts as an electrolyte and transfers the signal to the body. However, they dry out over time, and therefore, cannot be used for prolongated stimulations. The currently available electrodes are not often recommended for use longer than 24 hours. In addition, they do not have long storage times, most of the cases one month at the maximum after opening. In addition, they need a special packaging preventing them from drying out.

Many of the currently used gel electrodes can be used more than once to save material and costs, however the gel in the electrode gets dirty after several use, and this is not acceptable in a hospital environment and physiotherapy practices. Further, the high-water content is one reason why these gel electrodes tend to dry out. While the water content decreases, the signal quality also decreases, and therefore, the gel electrodes are not ideal for long-term use (maximum of three days).

Some of the stimulation electrodes currently on the market are attached to the skin via a gel-type adhesive. These electrodes do not need an additional skin adhesive since the gel itself is adhering to the skin. However, these electrodes also get dirty when used several times, which is not acceptable as mentioned above. In addition, this type of gel dries out over longer use. Solution to the drying problem has been to distribute some additional gel over the electrode area to improve the functionality.

Therefore, there is a need for electrodes for use in stimulation applications, which can be used for a week without loss of signal or adhesion while providing good current distribution and not drying out or sensitizing or irritating the skin. Documents US 2019/048193; US 6 121 508 and US 2018/086948 disclose known stimulation electrodes; WO 2020/180186 is prior art under Art. 54(3) EPC and also discloses a stimulation electrode according to the state of the art.

### Brief description of the figures

Figure 1 illustrates estimation of current distribution for high impedance adhesive and conductive collection layers with varying resistance.
Figure 2 illustrates estimation of current distribution for low impedance gel and conductive collection layers with varying resistance.
Figure 3 illustrates a simple resistance model for the estimation of the impact of the ratio of adhesive resistance to conductive layer resistance.
Figure 4 illustrates impedance spectra of the adhesive according to formula 3 on carbon contacting layer (55 Ohm/sq) for varying electrode dimensions (in cm).
Figure 5 illustrates impedance spectra of the adhesive according to formula 3 on carbon contacting layer (610 Ohm/sq) for varying electrode dimensions (in cm).
Figure 6 illustrates area scaled impedance spectra of the adhesive according to formula 3 on carbon contacting layer (55 Ohm/sq) for varying electrode dimensions (in cm).
Figure 7 illustrates area scaled impedance spectra of adhesive according to formula 3 on carbon contacting layer (610 Ohm/sq) for varying electrode dimensions (in cm).
Figure 8 illustrates the current variation based on a skin impedance variation for different electrode impedances (Ohm) (assuming that the typical skin impedance is 1E4 Ohm).
Figure 9 illustrates impedance spectra for various salts with 1-ethyl-3-methylimidazolium cation (Al contacts with sample area 0.5×0.5cm²).
Figure 10 illustrates impedance of electrodes according to example 4 using adhesive according to formula 3 on carbon contact (area 0.5×0.5cm²).
Figure 11 illustrates impedance of electrodes according to example 5 using adhesive according to formula 3 on Ag/AgCI contact (area 2×2cm²).
Figure 12 illustrates impedance of electrodes according to examples 6 and 7 comprising adhesive according to formula 11 and Ag/AgCI contact (full line) and carbon contact (slashed line) (area 2×2cm²).

### Summary of the invention

The present invention relates to a stimulation electrode comprising an ionically conductive pressure sensitive adhesive composition comprising a) a (meth)acrylate resin comprising at least 10% of a (meth)acrylate monomer comprising OH-group by weight of the total weight of the (meth)acrylate resin; and b) an ionic liquid.

### Detailed description of the invention

In the following passages the present invention is described in more detail. Each aspect so described may be combined with any other aspect or aspects unless clearly indicated to the contrary. In particular, any feature indicated as being preferred or advantageous may be combined with any other feature or features indicated as being preferred or advantageous.

In the context of the present invention, the terms used are to be construed in accordance with the following definitions, unless a context dictates otherwise.

As used herein, the singular forms "a", "an" and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps.

As used herein, the term *"consisting of"* excludes any element, ingredient, member or method step not specified.

The recitation of numerical end points includes all numbers and fractions subsumed within the respective ranges, as well as the recited end points.

All percentages, parts, proportions and then like mentioned herein are based on weight unless otherwise indicated.

When an amount, a concentration or other values or parameters is/are expressed in form of a range, a preferable range, or a preferable upper limit value and a preferable lower limit value, it should be understood as that any ranges obtained by combining any upper limit or preferable value with any lower limit or preferable value are specifically disclosed, without considering whether the obtained ranges are clearly mentioned in the context.

Unless otherwise defined, all terms used in disclosing the invention, including technical and scientific terms, have the meaning as commonly understood by one of the ordinary skilled in the art to which this invention belongs to. By means of further guidance, term definitions are included to better appreciate the teaching of the present invention.

A dry stimulation electrode according to the present invention comprises an ionically conductive pressure sensitive adhesive (PSA) with low impedance, good current distribution and good skin compatibility.

The present invention pertains to a stimulation electrode, which does not require a gel or hydrogel, therefore the term "dry electrode" is employed for the stimulation electrode according to the present invention as well.

By the term dry stimulation electrode is meant herein an electrode which does not contain a substantial amount of solvent and is free of water. The dry stimulating electrode according to the present invention comprises less than 1% of weight of the total weight of the adhesive a solvent, preferably less than 0.1% and more preferably less than 0.01%.

A dry stimulation electrode according to the present invention offers a solution to a long-term use in stimulating, wherein the long-term use is possible with stable impedance and adhesion. In contrast to the gel-type electrodes currently in the market it cannot dry out and it does not lead to skin irritation. The dry electrode according to the present invention has longer shelf-life and a storage of the electrodes is possible even after opening of the package. Further, the dry electrode according to the present invention provides comfort due to the very thin and dry layer leading to electrodes one does not feel in contrast to state of the art gel electrodes that are relatively heavy and swell the skin. In combination with thin, flexible and breathable substrates like polyurethane films the electrodes according to the present invention will allow a comfortable continuous long-term application.

The current distribution is of critical importance for the stimulation electrodes. A poor current distribution means that the usually high currents needed are fed into the body only via a small area close to the feed-in point on the electrode leading to high local current densities. A high current density may cause local pain at the feed-in point, whereas the part of the electrode which is far away from the feed-in point may only have decreased functionality due to lower current density. The current density is a main factor affecting the comfort of a stimulation.

The impedance of the adhesive or gel layer which is in a direct contact to the skin is one main factor for the current density. In general, the current distribution is improved by increasing impedance of the skin contact layer and decreasing impedance of the conductive layer underneath the skin contact layer. Standard gel adhesives have lower impedance than the dry adhesive according to the present invention. The lower impedance requires a highly conductive layer comprising high quality conductors such as Ag or Ag/AgCI to ensure similar current density over the whole electrode area. On the other hand, high impedance materials in the skin contact layer improve the current distribution but lead to Ohmic losses making the stimulation device (e.g. a TENS device) less efficient. In the dry stimulation electrode according to the present invention, the impedance of the skin contact layer is close to the skin impedance leading to an excellent compromise between low-loss contacting and good current distribution even when the electrode layers have quite low conductivities. Therefore, an ionically conductive pressure-sensitive adhesive according to the present invention can be combined with less conductive layers, e.g. carbon layers which are much cheaper than highly conductive Ag layers which are often used in stimulation electrodes with gel adhesives. The relatively high conductivity of the gel skin contact layer requires a highly conductive layer underneath to assure a good current distribution and thus a constant signal over the whole electrode area.

The ionically conductive pressure sensitive adhesive used in the present invention is based on a polar solvent-based acrylic pressure sensitive adhesive with high breathability and a non-toxic, non-irritating ionic liquid leading to an ionic conductivity and a good current distribution in the electrode while having a low sensitivity to skin inhomogeneities. In addition, the impedance of the PSA according to the present invention is sufficiently low without any addition of water preventing high Ohmic losses.

More specifically, the present invention refers to a dry stimulating electrode comprising a) an ionically conductive pressure sensitive adhesive composition comprising a (meth)acrylate resin comprising (meth)acrylate monomer comprising OH-group (hydroxyl group) and an ionic liquid; b) a conductive layer, which is in contact with said conductive pressure sensitive adhesive layer; c) optionally a substrate, which is in contact with the conductive layer; d) optionally a release liner, which is in contact with the conductive pressure sensitive adhesive layer; and e) optionally a contact between said electrode and stimulation device.

A dry stimulating electrode according to the present invention comprises an ionically conductive pressure sensitive adhesive.

An ionically conductive pressure sensitive adhesive composition according to the present invention comprises a (meth)acrylate resin comprising at least 10% of a (meth)acrylate monomer comprising OH-group by weight of the total weight of the (meth)acrylate resin.

Suitable (meth)acrylate resin for use in the present invention is preferably formed from the monomers selected from the group consisting of hydroxyethyl acrylate, hydroxypropyl acrylate, hydroxybutyl acrylate, methyl methacrylate, butyl acrylate, ethylhexylacrylate, acrylic acid, C1-C18 alkyl (meth)acrylate, (meth)acrylamide, vinyl acetate, N-vinyl caprolactame, acrylonitrile, vinyl ether, benzyl (meth)acrylate, cyclohexyl (meth)acrylate, glycidyl (meth)acrylate and mixtures thereof, preferably formed from the monomers selected from the group consisting of hydroxyethyl acrylate, methyl methacrylate, butyl acrylate, ethylhexylacrylate and mixtures thereof, and more preferably said (meth)acrylate resin is formed from hydroxyethyl acrylate, methyl (meth)acrylate, butyl acrylate and ethylhexylacrylate.

Suitable commercially available (meth)acrylate resins for use in the present invention include, but are not limited to Loctite DURO-TAK 222A, Loctite DURO-TAK 87-202A; Loctite DURO-TAK 87-402A; Loctite DURO-TAK 73-626A from Henkel.

The applicant has found out that a PSA comprising a (meth)acrylate resin comprising at least 10% of a (meth)acrylate monomer comprising OH-group provides good impedance and electrodes do not dry out and they can be used for longer period stimulation (the higher OH content increases the water vapor transmission rate of the polymer, which contributes to increased breathability and longer wear times).

Preferably content of said (meth)acrylate monomer comprising OH-group in said (meth)acrylate resin is at least 15% by weight of the total weight of the (meth)acrylate resin, preferably at least 20%, more preferably at least 25%, and most preferably at least 30%, but no more than 65%, preferably no more than 60%, more preferably no more than 55%, and most preferably no more than 50%.

When the (meth)acrylate monomer comprising OH-group in said (meth)acrylate resin is more than 65% by weight of the total weight of the (meth)acrylate resin, the higher OH-group content may negatively affect the adhesion properties.

An ionically conductive pressure sensitive adhesive composition used in a stimulation electrode according to the present invention may comprise said (meth)acrylate resin from 10 to 99% by weight of the total weight of the composition, preferably from 25 to 98% and more preferably from 50 to 95%.

Lower (meth)acrylate resin quantity may lead to poor adhesion properties and is not beneficial to film forming properties, whereas too high quantity may lead to poor conductivity.

An ionically conductive pressure sensitive adhesive composition according to the present invention comprises an ionic liquid, preferably a non-toxic, non-irritating ionic liquid leading to ionic conductivity.

More specifically, an ionically conductive pressure sensitive adhesive composition according to the present invention comprises an ionic liquid selected from the group consisting of imidazolium acetates, imidazolium sulfonates, imidazolium chlorides, imidazolium sulphates, imidazolium phosphates, imidazolium thiocyanates, imidazolium dicyanamides, imidazolium benzoates, imidazolium triflates, choline triflates, choline saccharinate, choline sulfamates, pyridinium acetates, pyridinium sulfonates, pyridinium chlorides, pyridinium sulphates, pyridinium phosphates, pyridinium thiocyanates, pyridinium dicyanamides, pyridinium benzoates, pyridinium triflates, pyrrolidinium acetates, pyrrolidinium sulfonates, pyrrolidinium chlorides, pyrrolidinium sulphates, pyrrolidinium phosphates, pyrrolidinium thiocyanates, pyrrolidinium dicyanamides, pyrrolidinium benzoates, pyrrolidinium triflates, phosphonium acetates, phosphonium sulfonates, phosphonium chlorides, phosphonium sulphates, phosphonium phosphates, phosphonium thiocyanates, phosphonium dicyanamides, phosphonium benzoates, phosphonium triflates, sulfonium acetates, sulfonium sulfonates, sulfonium chlorides, sulfonium sulphates, sulfonium phosphates, sulfonium thiocyanates, sulfonium dicyanamides, sulfonium benzoates, sulfonium triflates, ammonium acetates, ammonium sulfonates, ammonium chlorides, ammonium sulphates, ammonium phosphates, ammonium thiocyanates, ammonium dicyanamides, ammonium benzoates, ammonium triflates and mixtures thereof.

Preferably, said ionic liquid is selected from the group consisting of 1-ethyl-3-methylimidazolium acetate, 1-ethyl-3-methylimidazolium methane sulfonate, 1-ethyl-3-methylimidazolium trifluormethane sulfonate, 1-ethyl-3-methylimidazolium chloride, 1-ethyl-3-methylimidazolium ethyl sulphate, 1-ethyl-3-methylimidazolium diethylphosphate, 1-ethyl-3-methylimidazolium thiocyanate, 1-ethyl-3-methylimidazolium dicyanamide, 1-ethyl-3-methylimidazolium benzoate, choline trifluormethanesulfonate, choline saccharinate, choline acesulfamate, choline *N-*cyclohexylsulfamate, tris(2-hydroxyethyl)methylammonium methyl sulphate, 1-ethyl-3-methylimidazolium tetrafluoroborate, 1-allyl-3-methylimidazolium bis(trifluoromethylsulfonyl)imide, choline acetate and mixtures thereof.

More preferably, the ionic liquid is selected from the group consisting of 1-ethyl-3-methylimidazolium benzoate, 1-ethyl-3-methylimidazolium tetrafluoroborate, 1-ethyl-3-methylimidazolium methane sulfonate, 1-ethyl-3-methylimidazolium chloride, 1-ethyl-3-methylimidazolium trifluoromethane sulfonate, choline trifluoromethane sulfonate, 1-ethyl-3-methylimidazolium acetate, choline acetate, 1-ethyl-3-methylimidazolium diethylphosphate, 1-allyl-3-methylimidazolium bis(trifluoromethylsulfonyl)imide, 1-ethyl-3-methylimidazolium ethyl sulphate, 1-ethyl-3-methylimidazolium thiocyanate, 1-ethyl-3-methylimidazolium dicyanamide, choline saccharinate, choline acesulfamate, and mixtures thereof.

Above mentioned ionic liquids are preferred because they have good solubility into the (meth)acrylate resin according to the present invention and low toxicity.

Suitable commercially available ionic liquids for use in the present invention include, but are not limited to Basionics ST80, Basionics Kat1, Basionics BC01, Basionics VS11, Basionics VS03, and Efka IO 6785, all from BASF.

An ionically conductive pressure sensitive adhesive composition used in a stimulation electrode according to the present invention may comprise an ionic liquid from 0.1 to 50% by weight of the total weight of the composition, preferably from 1 to 35%, and more preferably from 4 to 25%.

If the quantity of the ionic liquid is too low, the adhesive may not show any ionic conductivity and the signal may be lost, whereas too high quantity may not provide improvement in a signal quality but may increase the chances of skin irritation and decrease the adhesion properties.

An ionically conductive pressure sensitive adhesive composition according to the present invention may further comprise an ionic conductivity promoter, preferably a non-toxic, non-irritating ionic conductivity promoter leading to additional ionic conductivity. The ionic conductivity promoter is preferably semi-solid or solid under room temperature and can be dissolved in the ionic liquid. It has good compatibility with the (meth)acrylate resin according to the present invention.

Preferably said ionic conductivity promoter is selected from the group consisting of choline chloride, choline bitartrate, choline dihydrogen citrate, choline phosphate, choline gluconate, choline fumarate, choline carbonate, choline pyrophosphate and mixtures thereof.

According to the present invention, the ionically conductive pressure sensitive adhesive composition may comprise an ionic conductivity promoter from 0.1 to 35% by weight of the total weight of the composition, preferably from 0.5 to 25%, and more preferably from 1 to 15%.

If the quantity of the ionic conductivity promoter is too low, the pressure sensitive adhesive may not show any ionic conductivity and the signal may be lost, whereas too high quantity may not provide improvement in signal quality but may increase the chances of skin irritation and decrease the adhesion properties.

An ionically conductive pressure sensitive adhesive composition according to the present invention may further comprise electrically conductive particles.

Preferably electrically conductive particles are selected from the group consisting of metal particles and metal nanoparticles, metal containing particles and nanoparticles, graphite particles and nanoparticles, carbon particles and nanoparticles, carbon nanowires, conductive polymer particles and nanoparticles, and mixtures thereof, more preferably selected from the group consisting of silver containing particles, silver particles, copper particles, copper containing particles, silver nanowires, copper nanowires, graphite particles, carbon particles and mixtures thereof, and even more preferably selected from graphite particles, carbon particles and mixtures thereof.

Graphite particles and carbon particles are preferred due to the fact that they do not cause skin irritation and are usually cheap especially compared to high conductive noble metal particles, however they provide adequate conductivity.

Suitable commercially available electrically conductive particles for use in the present invention include, but are not limited to Ensaco 250G, Timrex KS6 from Timcal, Printex XE2B from Necarbo, C-Nergy Super C65 from Imerys and Vulcan XC72R from Cabot.

An ionically conductive pressure sensitive adhesive composition used in a stimulation electrode according to the present invention may comprise said electrically conductive particles from 0.01 to 30% by weight of the total weight of the composition, preferably from 0.1 to 25%, and more preferably from 1 to 20%.

If the quantity of the electrically conductive particles is too low, it may lead to poor conductivity, whereas too high quantity may lead to loss of adhesion properties.

An ionically conductive pressure sensitive adhesive composition according to the present invention may further comprise a polyether polyol. Preferably, the polyether polyol is selected from polyethylene glycol (PEG), polypropylene glycol (PPG), polytetramethylene glycol (PTMG) and mixtures thereof. The applicant has found out that addition of polyether polyol is an exceptionally good host for ionic conductivity due to the open and flexible molecule chains, and therefore, has a positive impact on the impedance. The applicant has found out that already a small quantity of polyether polyol has a positive impact, which is beneficial regarding the skin compatibility of the composition.

Preferably, the polyether polyol may have a weight averaged molecular weight (Mw) from 300 to 1000 g/mol, preferably from 350 to 750 g/mol and more preferably from 380 to 420 g/mol, wherein the molecular weight is measured by gel permeation chromatography according to DIN 55672-1 :2007-08 with THF as the eluent.

Suitable commercially available polyether polyol for use in the present invention include but is not limited to Kollisolv PEG 400 from BASF.

An ionically conductive pressure sensitive adhesive composition used in a stimulation electrode according to the present invention may comprise polyether polyol from 0.01 to 50% by weight of the total weight of the composition, preferably from 0.1 to 25% and more preferably from 1 to 20%.

Too high polyether polyol quantity may lead to loss of adhesion properties.

An ionically conductive pressure sensitive adhesive composition according to the present invention may further comprise a solvent.

Suitable solvent for use in the present invention may be selected from the group consisting of water, ethyl acetate, butyl acetate, butyl diglycol, 2-butoxyethanol, ethylene glycol, diethylene glycol, propylene glycol, dipropylene glycol, methanol, isopropanol, butanol, dibasic esters, hexane, heptane, 2,4-pentadione, toluene, xylene, benzene, hexane, heptane, methyl ethyl ketone, methyl isobutyl ketone, diethylether and mixtures thereof, preferably said solvent is selected from the group consisting of ethyl acetate, butyl acetate, ethylene glycol, propylene glycol and mixtures thereof.

Suitable commercially available solvents for use in the present invention include but are not limited to ethyl acetate and ethylene glycol from Brenntag, butyl acetate from Shell Chemicals and propylene glycol from Lyondell.

An ionically conductive pressure sensitive adhesive composition used in a stimulation electrode according to the present invention may comprise a solvent from 10 to 90% by weight of the total weight of the composition, preferably from 20 to 80%, and more preferably from 30 to 70%.

If the quantity of the solvent is too low, this may lead to processability problems due to the fact that the viscosity is too high, and (meth)acrylate resin may not be fully soluble. Whereas too high quantity may lead to loss of functionality, and the viscosity of the adhesive is too low to process.

For the sake of the clarity, it is noted that the pressure sensitive adhesive according to the present invention comprises a solvent. A pressure-sensitive adhesive film formed from said adhesive is used in the dry stimulating electrode according to the present invention and the film does not comprise a substantial amount of solvent. As described above the dry stimulating electrode according to the present invention comprises less than 1% of weight of the total weight of the adhesive a solvent, preferably less than 0.1% and more preferably less than 0.01%.

An ionically conductive pressure sensitive adhesive composition according to the present invention ideally has an impedance in a similar range than the skin impedance. The impedance depends on the area of the electrode and underlying skin.

An ionically conductive pressure sensitive adhesive composition according to the present invention has impedance value below 1,000,000 Ohm at 1000 Hz, but more than 1000 Ohm at 1000 Hz, preferably below 100,000 Ohm at 1000 Hz, but more than 5000 Ohm at 1000 Hz and more preferably below 40,000 Ohm at 1000Hz but more than 8000 Ohm at 1000 Hz, wherein said impedance is measured by connecting two electrodes coated each with 25 µm of an ionic conductive pressure sensitive adhesive having a contact area of 0.25 cm².

The combination of the (meth)acrylate resin and the ionic liquid leads to a low impedance in the pressure sensitive adhesive according to the present invention. The ionic liquid provides the ionic conductivity, however, if the ionic liquid is not miscible with the (meth)acrylate resin, one will not see any ionic conductivity in the pressure sensitive adhesive. In the embodiment, wherein PEG is added to the composition, the additional ether groups from the PEG make the system more polar and enhance the ionic conductivity of the ionic liquid in the (meth)acrylate resin.

An ionically conductive pressure sensitive adhesive composition according to the present invention has high breathability. Good breathability is obtained if the water can penetrate easily through the adhesive layer. To achieve this effect, a quite polar resin is required, in this occasion, the OH-functionalities support and improve the breathability.

Adhesive according to the present invention has preferably a breathability value of about 4600 g/m² in 24 hours. As a comparison a standard acrylic PSA has a breathability value of about 2000 g/m² in 24 hours. The breathability is measured through a moisture vapor transmission rate (MVTR) measurement according to ASTM D1653.

The present invention relates to a dry stimulation electrode formed from the ionically conductive pressure sensitive adhesive composition as described above. The dry film formation can be done by coating the ionically conductive pressure sensitive adhesive composition on a supporting substrate (such as a film) and drying the film in an oven at for example 120°C for 3 minutes to remove the solvent and form a dry film of the ionically conductive pressure sensitive adhesive on the supporting substrate.

The known method used for preparing pressure-sensitive adhesive can be used. Specifically, examples include roll coating, gravure coating, reverse coating, roll brushing, spray coating, and air knife coating methods, immersing and curtain coating method, and extruding coating method with a die coater.

A stimulation electrode according to the present invention further comprises b) a conductive layer, which is in contact with said ionically conductive pressure sensitive adhesive layer; c) optionally a substrate, which is in contact with the conductive layer; d) optionally a release liner, which is in contact with the ionically conductive pressure sensitive adhesive layer; and e) optionally a contact between said electrode and stimulation device.

The electrode according to the present invention contains a conductive layer, preferably only one conductive layer.

In one embodiment the conductive layer is selected from copper, silver, gold, aluminium, Ag/AgCI, or a carbon layer or mixtures thereof.

In another embodiment the conductive layer has a thickness of 0.1 to 500 µm, or 0.5 to 150 µm, or 1 to 25 µm, or 1 to 20 µm.

In one embodiment, the conductive layer is a metal, preferably with a thickness of 10 to 500 µm, or 25 to 150 µm. Preferably, the metal is a copper, silver, gold, or aluminium layer.

It is important that the current is homogeneously distributed over the electrode area in order to obtain good functionality for the electrode. An inhomogeneous current distribution leads to areas with high current density that could potentially lead to a local pain or burns caused by high electrical stimulus at the contact point and areas with low current density with decreased stimulation functionality.

A homogeneous current distribution can be obtained if the ratio of impedance of conductive layer to impedance of ionically conductive pressure sensitive adhesive layer is small. The fact that the ionically conductive pressure sensitive adhesive according to the present invention shows a higher impedance than a gel adhesive means that a more homogeneous current distribution is obtained using the same conductive layer. The current calculated 2 cm apart from the feed-in point of a stimulation electrode with a gel adhesive having an impedance of 20 Ohm is only 10% of the current at the feed-in point for a conductive layer with a conductivity of 100 Ohm. A similar electrode with a 2 kOhm pressure-sensitive adhesive provides a relative current of 90% at a distance of 2 cm from the feed-in point (see example 1). Therefore, an ionically conductive pressure-sensitive adhesive having higher impedance than standard gel adhesives can be combined with conductive layers with lower conductivities and still allow good current distribution. Ionically conductive pressure sensitive adhesives do not need highly conductive layers (e.g. Ag or Cu layers) which are usually much more expensive than less conductive layers formed from carbon inks for example.

In a further embodiment the conductive layer is the only conductive layer contained in the electrode in addition to the ionically conductive pressure sensitive adhesive.

In preferred embodiments, the electrode according to the present invention contains a substrate. In one embodiment the substrate is a flexible film, preferably selected from polyolefin films, polycarbonate films, thermoplastic polyurethane (TPU) films, silicone films, woven films, non-woven films, or paper films, in particular polyethylene films, polypropylene films, polyethylene terephthalate films or thermoplastic polyurethane films or foams such as polyurethane foams, polyolefin foams and polycarbonate foams.

In another embodiment the substrate has a thickness of 10 to 500 µm, or 25 to 150 µm.

In order to package the electrode and avoid that the adhesive layer sticks to the package, the electrode may contain a release liner on the surface of the adhesive layer which is later applied to the area which should be measured. All known release liners in the art are suitable, in one embodiment the release liner is selected from siliconized paper release liner or plastic release liner.

As already stated above the electrode of the present invention does not require a gel/hydrogel. Therefore, in one embodiment, the electrode is essentially free from a hydrogel, preferably does not contain more than 0.5 wt.-%, or 0.1 wt.-%, or 0.01 wt.-% of a hydrogel, or does not contain a hydrogel, based on the total weight of the electrode.

In another embodiment the electrode is essentially free from an aqueous electrolyte paste, preferably does not contain more than 0.5 wt.-%, or 0.1 wt.-%, or 0.01 wt.-% of an aqueous electrolyte paste, or does not contain an aqueous electrolyte paste, based on the total weight of the electrode.

A stimulation electrode according to the present invention is used in skin applications transferring an electrical signal to the body via skin. Suitable applications are for example transcutaneous electrical nerve stimulation (TENS), electrical muscle stimulation (EMS), functional electrical stimulation (FES), wound care, electrosurgery, defibrillation, electrodermal activity and total body electrical conductivity.

It is preferred that the relative vitality of the cells in the epidermis test for the dry stimulation electrode adhesive composition according to the present invention is more than 50%, preferably more than 60% and more preferably more than 70%.

### Examples

### Example 1

For best functionality of a stimulation electrode following three conditions need to be met: 1) Ohmic losses cannot be too high; 2) the electrode has a good current distribution; and 3) low sensitivity to skin inhomogeneities.

Preventing Ohmic losses is not considered too critical, because most of the cases high Ohmic loss can be compensated by increasing the voltage. However, to get significant signal delivered to the body, the impedance of the electrode should not be much higher than the skin impedance. This usually applies for gel electrode adhesives and ionically conducting pressure sensitive adhesives as described in the description above.

The current distribution within the electrode will be influenced by the area (or the maximum distance to the feed-in point within the electrode) and the ratio of the resistances of the current collecting layer (typically metal or carbon) and the adhesive (or hydrogel as standardly used).

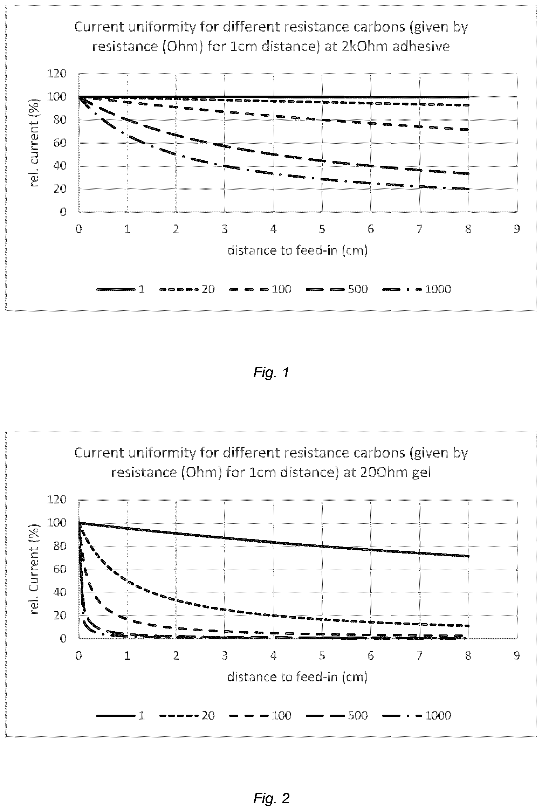

Figure 1 illustrates estimation of current distribution for high impedance adhesive and varying conductive collection layers. Current uniformity is illustrated for carbon layers with different resistances (given by resistance (Ohm) for 1 cm distance) at 2kOhm adhesive.

Figure 2 illustrates estimation of current distribution for low impedance gel and varying conductive collection layers. Current uniformity is illustrated for carbon layers with different resistances (given by resistance (Ohm) for 1 cm distance) at 20Ohm gel.

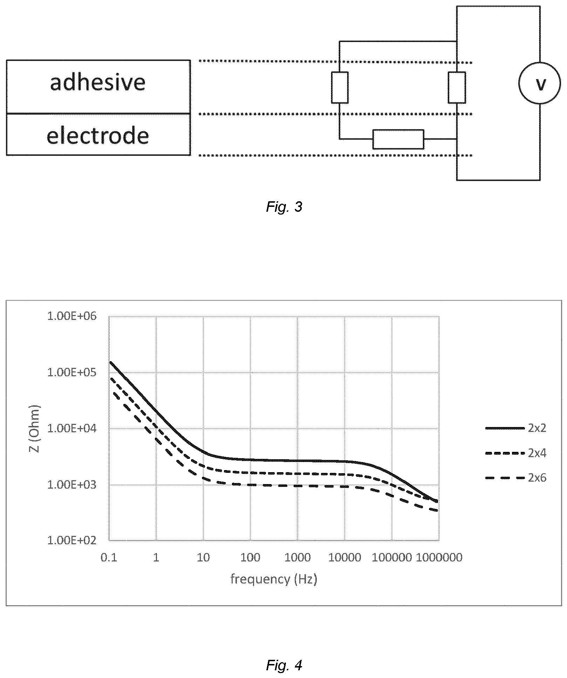

Figure 3 illustrates a simple resistance model for estimating the impact of adhesive/collector resistances.

In summary, figures 1 and 2 illustrate the current variation depending on the resistance of the conductive layer calculated using the simple model from figure 3 assuming a relatively low conductive adhesive (roughly corresponding to formula 3 described in example 2 below) and a high conductive gel, respectively. It is clear that using a relatively low conductive adhesive a good current distribution can be achieved using only moderately conductive electrodes. This has been experimentally confirmed with real electrodes. The conductive adhesive used in these experiments had an Ohmic plateau of about 2k Ohm (at 2x2 cm²) and corresponds to formula 3.

Figure 4 illustrates an impedance spectrum of the adhesive according to formula 3 on carbon contacting layer (55 Ohm/sq) for varying electrode dimensions (in cm). Contacting of the samples was on the short side.

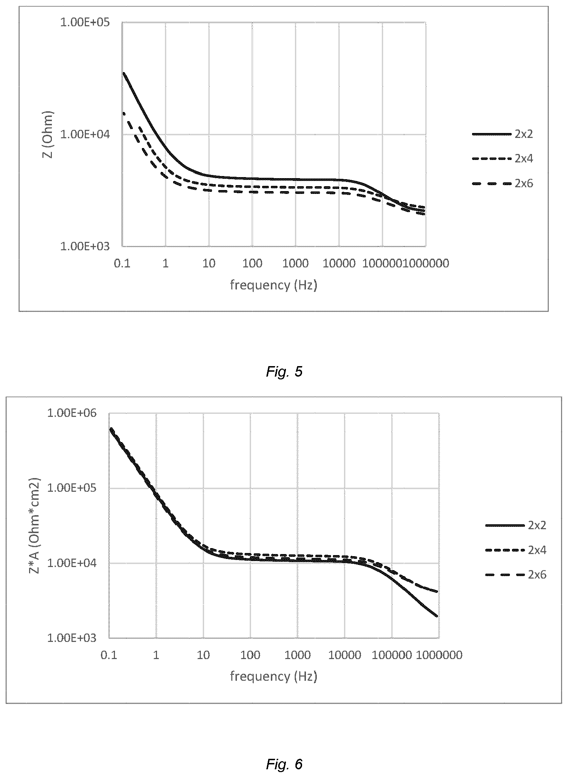

Figure 5 illustrates an impedance spectrum of the adhesive according to formula 3 on carbon contacting layer (610 Ohm/sq) for varying electrode dimensions (in cm). Contacting of the samples was on the short side.

Figure 4 displays the impedance spectra of an adhesive on carbon contacts for varying electrode sizes. The sheet resistance is 55 Ohm/sq, which corresponds to a relatively bad contact (low conductance). The impedance was reduced for increasing electrode area as expected. Figure 5 shows the same curve for a contact layer which has been made as low conductive as possible (610 Ohm/sq). Similarly, the impedance was reduced for increasing electrode areas. Note however that the difference was smaller than for the higher conductive contact layer. To achieve a perfect current distribution, the impedance should be scaled with the inverse of the area, and therefore, Z*A should be constant.

Figure 6 illustrates an area scaled impedance spectrum of adhesive according to formula 3 below on carbon contacting layer (55 Ohm/sq) for varying electrode dimension (in cm). Contacting of the samples was on the short side.

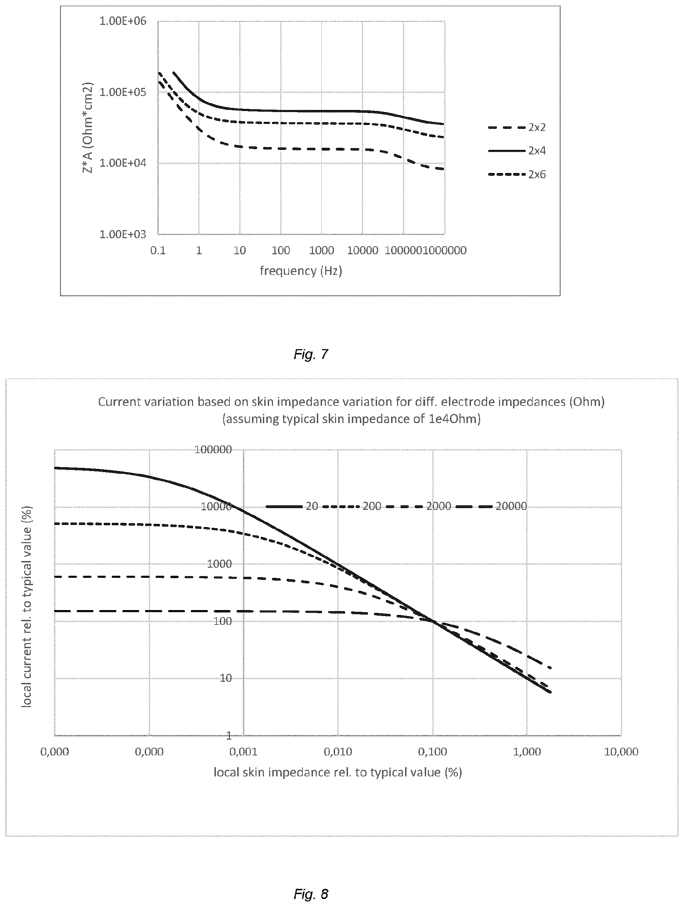

Figure 7 illustrates an area scaled impedance spectrum of adhesive according to formula 3 below on carbon contacting layer (610 Ohm/sq) for varying electrode dimension (in cm). Contacting of the samples was on the short side.

Both figures 6 and 7 illustrate the corresponding scaled impedance spectra. For the lower sheet resistance of the conductive layer the area scaling was perfect (except the high frequency range where the adhesive impedance becomes neglectable). For the high sheet resistance sample the majority of the current flew close to the contact (while the rest still contributed). Therefore, even with a moderate sheet resistance of 55 Ohm/sq an extremely good current distribution could be achieved. It is noted that an adhesive providing even worse conductivity could be used, therefore, 55 Ohm/sq is not considered as a limit.

The skin itself may have some inhomogeneities. Therefore, low resistive spots or areas should not lead to high local current densities and subsequent pain/damage.

Figure 8 illustrates the current variation based on a skin impedance variation for different electrode impedances (Ohm) (assuming that the typical skin impedance is 1E4 Ohm).

Figure 8 clearly illustrates how the local current can vary based on local variation of the skin impedance for different electrode adhesive impedances. Clearly, skin inhomogeneity becomes less critical the higher the electrode impedance is.

Based on the above three considerations electrodes should be designed in the following way:
- Use adhesive having a resistance similar or slightly higher than skin.
- Use an electrode material having significantly lower resistance (which does not need to be too low since adhesive resistance is quite high). Therefore, moderately conductive low-cost carbon inks may be used.
- Use adhesive based on ionic liquids for easy fine tuning and long wearing times. Choose an adhesive and an ionic liquid which are bio-compatible.

### Example 2

### Preparation of ionically conductive pressure sensitive conductive adhesive according to the present invention:

5 g Loctite Duro-Tak 222A and 0.228 g of ionic liquid were mixed in a conditioning mixer for 1 minute at 1000 rpm. The mixture was coated onto a release liner and dried at room temperature in the fumehood for 30 min yielding a PSA film with a thickness of 20-30 µm. Subsequently, the drawdown was cured at 120°C for 3 min and covered with another release liner.

For impedance measurements the PSA film was coated onto an Al foil, dried at RT for 30 min and cured at 120°C for 3 min. Two pieces of the PSA-AI film were cut and stuck together to form an Al-PSA-AI capacitor with an area of 0.25 cm² and 40-60 µm PSA thickness.

### Skin compatibility study:

The skin compatibility of the pressure sensitive adhesive composition was measured in an in-vitro skin irritation test using an OS-REp model (Open Source Reconstructed Epidermis). 25 µL of the active pressure sensitive adhesive composition was applied to the epidermis model. After 42 hours incubation and 3 hours incubation with MTT (200 µl 1 mg/ml MTT (3-(4,5-Dimethylthiazol-2-yl)-2,5-diphenyltetrazolium bromide)), a formazan extraction was performed and the optical density at 570-590 nm was measured. The relative vitality of the cells was calculated by the optical density.

The skin irritation test is a modified version of an OS-REp test according to OECD TG 439 which is a protocol for the identification of irritant neat substances and salts.

The usual contact time of the potential irritant with the skin model is 35 minutes. Subsequently the substance to be tested will be washed off and an incubation time of 42 hours starts. For a relative vitality of the cells >50% the substance can be considered being non-irritant.

Due the fact that pressure-sensitive adhesives were tested, which cannot be washed off, the contact time was significantly longer - 42 hours instead to 35 minutes - indicating that the present test conditions were harsher.

Details of the specific examples 1-10 and the test results regarding adhesion and epidermis test are listed in table 1 below.

**Table 1**

| *Example* | *Ionic liquid* | *Adhesion* | *Vitality* (% *according to epidermis test)* |
|---|---|---|---|
| Formula 1 | 1-Ethyl-3-methylimidazolium acetate | poor | 84 |
| Formula 2 | 1-Ethyl-3-methylimidazolium methanesulfonate | good | n.d. |
| Formula 3 | 1-Ethyl-3-methylimidazolium trifluormethanesulfonate | good | 91 |
| Formula 4 | 1-Ethyl-3-methylimidazolium chloride | good | n.d |
| Formula 5 | 1-Ethyl-3-methylimidazolium ethylsulfate | good | 63 |
| Formula 6 | 1-Ethyl-3-methylimidazolium diethylphosphate | good | n.d. |
| Formula 7 | 1-Ethyl-3-methylimidazolium thiocyanate | good | 95 |
| Formula 8 | 1-Ethyl-3-methylimidazolium dicyanamide | good | 80 |
| Formula 9 | 1-Ethyl-3-methylimidazolium benzoate | good | 78 |
| Formula 10 | Choline acetate | poor | n.d. |

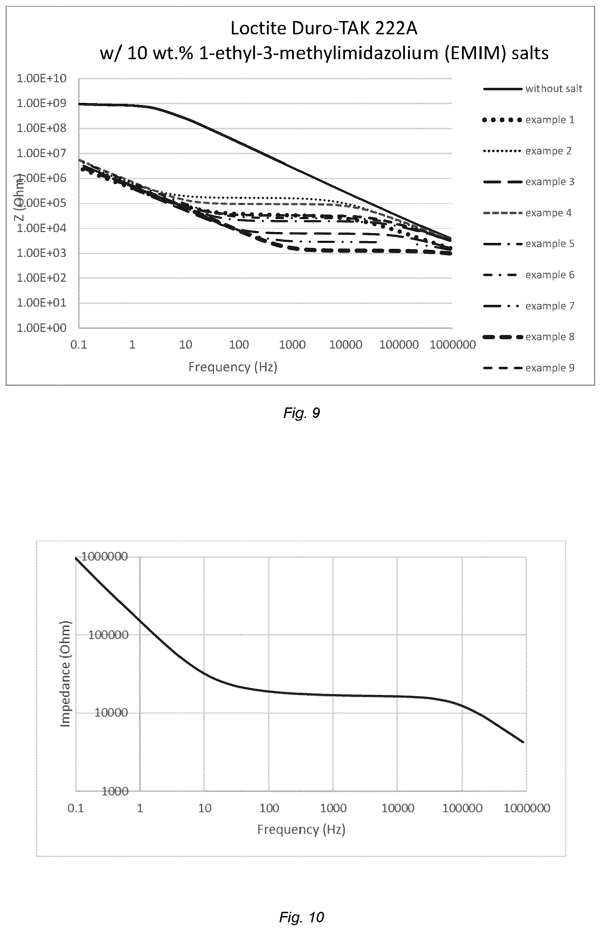

Figure 9 shows impedance measurements of dry PSA films according to formula 3 between aluminium electrodes (approximately 40-60 µm thickness, 0.25cm² electrode area). By using 10 wt% of the respective imidazolium salt, the impedance of the skin adhesive decreased significantly to values which are ideally suited for electrode applications compared to the adhesive without salt addition. While the choice of ionic liquid was the main driver for the impedance level which can be achieved, the impedance can also be finetuned by varying the concentration of the ionic liquid. This and by adapting the thickness, an adhesive can be chosen that matches a value similar to the skin impedance.

### Example 3

### Preparation of ionically conductive pressure sensitive adhesive with combination of ionic liquids (Formula 11).

5 g Loctite Duro-TAK 222A (solid content: 41%) and 0.171 g of 1-ethyl-3-methylimidazolium triflate and 0.057 g of 1-ethyl-3-methylimidazolium chloride were mixed in a conditioning mixer for 3 minutes at 2000 rpm. The mixture was coated onto a release liner and dried at room temperature for 30 min yielding PSA films with a thickness of 20 µm. Subsequently, the drawdown was cured at 120°C for 3 min and covered with another release liner.

### Example 4

### Preparation of a stimulating carbon-PSA electrode using formula 3

A conductive carbon layer with a thickness of around 10 µm was coated onto a flexible TPU film using LOCTITE ECI 7005 E&C from Henkel and dried for 10 min at 120°C and cut into rectangular pieces of 2 cm × 3 cm. A conductive PSA film (formula 3, 2 cm × 2 cm) was transferred to the rectangular pieces. For impedance measurements, smaller electrodes with an adhesive area of 0,5x0,5cm were prepared in the same manner. Two electrodes were stuck together with their complete adhesive area and connected with alligator clips. Figure 10 illustrates the impedance spectrum of the electrodes. For the application as stimulating electrodes two electrodes were stuck to the forearm with a distance of 10 cm from each other and connected to a "Sanitas SEM 43" TENS/EMS device. Electrical stimuli from the device led to clearly visible muscle contractions.

### Example 5

### Preparation of a stimulating Ag/AgCI-PSA electrode using formula 3

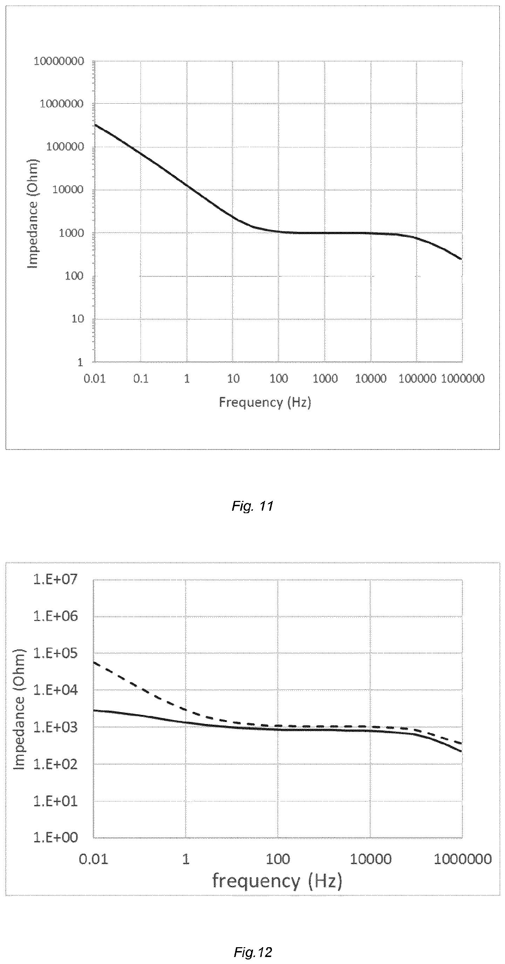

An Ag/AgCI layer with a thickness of around 10µm was coated onto a flexible TPU film using Loctite EDAG 6038E SS E&C from Henkel and cut into rectangular pieces of 2 cm × 3 cm. A conductive PSA film (Example 11, 2 cm × 2 cm) was transferred to the rectangular pieces. For impedance measurements two electrodes were stuck together with their complete adhesive area and connected with alligator clips. Figure 11 illustrates the impedance spectrum. For the application as stimulating electrodes two electrodes were stuck to the forearm with a distance of 10 cm from each other and connected to a "Sanitas SEM 43" TENS/EMS device. Electrical stimuli from the device led to clearly visible muscle contractions.

### Example 6

### Preparation of a stimulating carbon-PSA electrode using formula 11

A conductive carbon layer with a thickness of around 10 µm was coated onto a flexible TPU film using LOCTITE ECI 7005 E&C from Henkel and dried for 10 min at 120°C and cut into rectangular pieces of 2 cm × 3 cm. A conductive PSA film (formula 3, 2 cm × 2 cm) was transferred to the rectangular pieces. For impedance measurements two electrodes were stuck together with their complete adhesive area and connected with alligator clips. Figure 12 (dashed line) illustrates the impedance spectrum. For the application as stimulating electrodes two electrodes were stuck to the forearm with a distance of 10 cm from each other and connected to a "Sanitas SEM 43" TENS/EMS device. Electrical stimuli from the device led to clearly visible muscle contractions.

### Example 7

### Preparation of a stimulating Ag/AgCI-PSA electrode using formula 11

An Ag/AgCI layer with a thickness of around 10µm was coated onto a flexible TPU film using Loctite EDAG 6038E SS E&C from Henkel and cut into rectangular pieces of 2 cm × 3 cm. A conductive PSA film (Example 11, 2 cm × 2 cm) was transferred to the rectangular pieces. For impedance measurements two electrodes were stuck together with their complete adhesive area and connected with alligator clips. Figure 12 (solid line) illustrates the impedance spectrum. For the application as stimulating electrodes two electrodes were stuck to the forearm with a distance of 10 cm from each other and connected to a "Sanitas SEM 43" TENS/EMS device. Electrical stimuli from the device led to clearly visible muscle contractions. The present invention is set out in the claims that follow.

## Claims

1. A stimulation electrode comprising an ionically conductive pressure sensitive adhesive composition comprising
a) a (meth)acrylate resin comprising at least 10% of a (meth)acrylate monomer comprising OH-group by weight of the total weight of the (meth)acrylate resin; and
b) an ionic liquid,
wherein the stimulation electrode comprises less than 1% of weight of the total weight of the adhesive a solvent, is free of water and does not contain a hydrogel.

2. A stimulation electrode according to claim 1, wherein said (meth)acrylate resin formed from the monomers selected from the group consisting of hydroxyethyl acrylate, hydroxypropyl acrylate, hydroxybutyl acrylate, methyl methacrylate, butyl acrylate, ethylhexylacrylate, acrylic acid, C1-C18 alkyl (meth)acrylate, (meth)acrylamide, vinyl acetate, N-vinyl caprolactame, acrylonitrile, vinyl ether, benzyl (meth)acrylate, cyclohexyl (meth)acrylate, glycidyl (meth)acrylate and mixtures thereof, preferably formed from the monomers selected from the group consisting of hydroxyethyl acrylate, methyl methacrylate, butyl acrylate, ethylhexylacrylate and mixtures thereof, and more preferably said (meth)acrylate resin is formed from hydroxyethyl acrylate, methyl (meth)acrylate, butyl acrylate and ethylhexylacrylate.

3. A stimulation electrode according to claim 1 or 2, wherein said (meth)acrylate resin is present from 10 to 99% by weight of the total weight of the composition, preferably from 25 to 98% and more preferably from 50 to 95%.

4. A stimulation electrode according to any of claims 1 to 3, wherein the ionic liquid is selected from the group consisting of imidazolium acetates, imidazolium sulfonates, imidazolium chlorides, imidazolium sulphates, imidazolium phosphates, imidazolium thiocyanates, imidazolium dicyanamides, imidazolium benzoates, imidazolium triflates, choline triflates, choline saccharinate, choline sulfamates, pyridinium acetates, pyridinium sulfonates, pyridinium chlorides, pyridinium sulphates, pyridinium phosphates, pyridinium thiocyanates, pyridinium dicyanamides, pyridinium benzoates, pyridinium triflates, pyrrolidinium acetates, pyrrolidinium sulfonates, pyrrolidinium chlorides, pyrrolidinium sulphates, pyrrolidinium phosphates, pyrrolidinium thiocyanates, pyrrolidinium dicyanamides, pyrrolidinium benzoates, pyrrolidinium triflates, phosphonium acetates, phosphonium sulfonates, phosphonium chlorides, phosphonium sulphates, phosphonium phosphates, phosphonium thiocyanates, phosphonium dicyanamides, phosphonium benzoates, phosphonium triflates, sulfonium acetates, sulfonium sulfonates, sulfonium chlorides, sulfonium sulphates, sulfonium phosphates, sulfonium thiocyanates, sulfonium dicyanamides, sulfonium benzoates, sulfonium triflates, ammonium acetates, ammonium sulfonates, ammonium chlorides, ammonium sulphates, ammonium phosphates, ammonium thiocyanates, ammonium dicyanamides, ammonium benzoates, ammonium triflates and mixtures thereof.

5. A stimulation electrode according to any of claims 1 to 4, wherein said ionic liquid is selected from the group consisting of 1-ethyl-3-methylimidazolium acetate, 1-ethyl-3-methylimidazolium methanesulfonate, 1-ethyl-3-methylimidazolium trifluoromethanesulfonate, 1-ethyl-3-methylimidazolium chloride, 1-ethyl-3-methylimidazolium ethyl sulphate, 1-ethyl-3-methylimidazolium diethylphosphate, 1-ethyl-3-methylimidazolium thiocyanate, 1-ethyl-3-methylimidazolium dicyanamide, 1-ethyl-3-methylimidazolium benzoate, choline trifluoromethanesulfonate, choline saccharinate, choline acesulfamate, choline N-cyclohexylsulfamate, tris(2-hydroxyethyl)methylammonium methyl sulphate, 1-ethyl-3-methylimidazolium tetrafluoroborate, 1-allyl-3-ethylimidazolium bis(trifluoromethylsulfonyl)imide, choline acetate, choline trifluoromethanesulfonate and mixtures thereof, and more preferably selected from the group consisting of 1-ethyl-3-methylimidazolium benzoate, 1-ethyl-3-methylimidazolium tetrafluoroborate, 1-ethyl-3-methylimidazolium methanesulfonate, 1-ethyl-3-methylimidazolium chloride, 1-ethyl-3-methylimidazolium trifluoromethanesulfonate, choline trifluoromethanesulfonate, 1-ethyl-3-methylimidazolium acetate, choline acetate, 1-ethyl-3-methylimidazolium diethylphosphate, 1-allyl-3-methylimidazolium bis(trifluoromethylsulfonyl)imide, 1-ethyl-3-methylimidazolium ethyl sulphate, 1-ethyl-3-methylimidazolium thiocyanate, 1-ethyl-3-methylimidazolium dicyanamide, choline saccharinate, choline acesulfamate, and mixtures thereof.

6. A stimulation electrode according to any of claims 1 to 5, wherein said ionic liquid is present from 0.1 to 50% by weight of the total weight of the composition, preferably from 1 to 35%, and more preferably from 4 to 25%.

7. A stimulation electrode according to any of claims 1 to 6, wherein the content of said (meth)acrylate monomer comprising OH-group in said (meth)acrylate resin is at least 15% by weight of the total weight of the (meth)acrylate resin, preferably at least 20%, more preferably at least 25%, and most preferably at least 30%, but no more than 65%, preferably no more than 60%, more preferably no more than 55%, and most preferably no more than 50%.

8. A stimulation electrode according to any of claims 1 to 7, further comprising an ionic conductivity promoter, preferably selected from the group consisting of choline chloride, choline bitartrate, choline dihydrogen citrate, choline phosphate, choline gluconate, choline fumarate, choline carbonate, choline pyrophosphate and mixtures thereof.

9. A stimulation electrode according to claim 8, wherein said ionic conductivity promoter is present from 0.1 to 35% by weight of the total weight of the composition, preferably from 0.5 to 25%, and more preferably from 1 to 15%.

10. A stimulation electrode according to any of claims 1 to 9, further comprising electrically conductive particles, preferably selected from the group consisting of metal particles and metal nanoparticles, metal containing particles and nanoparticles, graphite particles and nanoparticles, carbon particles and nanoparticles, carbon nanowires, conductive polymer particles and nanoparticles, and mixtures thereof, more preferably selected from the group consisting of silver containing particles, silver particles, copper particles, copper containing particles, silver nanowires, copper nanowires, graphite particles, carbon particles and mixtures thereof, and even more preferably selected from graphite particles, carbon particles and mixtures thereof.

11. A stimulation electrode according to claim 10, wherein said electrically conductive particles are present from 0.01 to 30% by weight of the total weight of the composition, preferably from 0.1 to 25%, and more preferably from 1 to 20%.

12. A stimulation electrode according to any of claims 1 to 11, further comprising a polyether polyol, preferably selected from polyethylene glycol, polypropylene glycol, polytetramethylene glycol, and more preferably polyethylene glycol having weight averaged molecular weight from 300 to 1000, more preferably from 350 to 750 and even more preferably from 380 to 420 g/mol, wherein the molecular weight is measured by gel permeation chromatography according to DIN 55672-1:2007-08 with THF as the eluent.

13. A stimulation electrode according to claim 12, wherein said polyether polyol is present from 0.01 to 50% by weight of the total weight of the composition, preferably from 0.1 to 25%, and more preferably from 1 to 20%.

14. A stimulation electrode according to any of claims 1 to 13, wherein said adhesive has impedance value below 1,000,000 Ohm at 1000 Hz, but more than 1000 Ohm at 1000 Hz, preferably below 100,000 Ohm at 1000 Hz, but more than 5000 Ohm at 1000 Hz and more preferably below 40,000 Ohm at 1000Hz but more than 8000 Ohm at 1000 Hz, wherein said impedance is measured by connecting two electrodes coated each with 25 µm of an ionic conductive pressure sensitive adhesive having a contact area of 0.25 cm².

15. A stimulation electrode according to any of claims 1 to 14, wherein said electrode further comprises b) a conductive layer, which is in contact with said ionically conductive pressure sensitive adhesive layer; c) optionally a substrate, which is in contact with the conductive layer; d) optionally a release liner, which is in contact with the conductive pressure sensitive adhesive layer; and e) optionally a contact between said electrode and stimulation device.

## Patentansprüche

1. Stimulationselektrode, umfassend eine ionisch leitfähige, druckempfindliche Klebstoffzusammensetzung, umfassend
a) ein (Meth)acrylatharz, umfassend mindestens zu 10 Gew.-% des Gesamtgewichts des (Meth)acrylatharzes ein (Meth)acrylatmonomer, umfassend eine OH-Gruppe; und
b) eine ionische Flüssigkeit,
wobei die Stimulationselektrode weniger als zu 1 Gew.-% des Gesamtgewichts des Klebstoffs, ein Lösungsmittel umfasst, wasserfrei ist und kein Hydrogel enthält.

2. Stimulationselektrode nach Anspruch 1, wobei das (Meth)acrylatharz aus den Monomeren ausgebildet ist, die aus der Gruppe ausgewählt sind, bestehend aus Hydroxyethylacrylat, Hydroxypropylacrylat, Hydroxybutylacrylat, Methylmethacrylat, Butylacrylat, Ethylhexylacrylat, Acrylsäure, C1-C18-Alkyl(meth)acrylat, (Meth)acrylamid, Vinylacetat, N-Vinylcaprolactam, Acrylnitril, Vinylether, Benzyl(meth)acrylat, Cyclohexyl(meth)acrylat, Glycidyl(meth)acrylat und Gemischen davon, vorzugsweise aus den Monomeren ausgebildet ist, die aus der Gruppe ausgewählt sind, bestehend aus Hydroxyethylacrylat, Methylmethacrylat, Butylacrylat, Ethylhexylacrylat und Gemischen davon, und stärker bevorzugt das (Meth)acrylatharz aus Hydroxyethylacrylat, Methyl(meth)acrylat, Butylacrylat und Ethylhexylacrylat ausgebildet ist.

3. Stimulationselektrode nach Anspruch 1 oder 2, wobei das (Meth)acrylatharz von 10 bis 99 Gew.-% des Gesamtgewichts der Zusammensetzung, vorzugsweise von 25 bis 98 Gew.-% und stärker bevorzugt von 50 bis 95 Gew.-% vorhanden ist.

4. Stimulationselektrode nach einem der Ansprüche 1 bis 3, wobei die ionische Flüssigkeit aus der Gruppe ausgewählt ist, bestehend aus Imidazoliumacetaten, Imidazoliumsulfonaten, Imidazoliumchloriden, Imidazoliumsulfaten, Imidazoliumphosphaten, Imidazoliumthiocyanaten, Imidazoliumdicyanamiden, Imidazoliumbenzoaten, Imidazoliumtriflaten, Cholintriflaten, Cholinsaccharinat, Cholinsulfamaten, Pyridiniumacetaten, Pyridiniumsulfonaten, Pyridiniumchloriden, Pyridiniumsulfaten, Pyridiniumphosphaten, Pyridiniumthiocyanaten, Pyridiniumdicyanamiden, Pyridiniumbenzoaten, Pyridiniumtriflaten, Pyrrolidiniumacetaten, Pyrrolidiniumsulfonaten, Pyrrolidiniumchloriden, Pyrrolidiniumsulfaten, Pyrrolidiniumphosphaten, Pyrrolidiniumthiocyanaten, Pyrrolidiniumdicyanamiden, Pyrrolidiniumbenzoaten, Pyrrolidiniumtriflaten, Phosphoniumacetaten, Phosphoniumsulfonaten, Phosphoniumchloriden, Phosphoniumsulfaten, Phosphoniumphosphaten, Phosphoniumthiocyanaten, Phosphoniumdicyanamiden, Phosphoniumbenzoaten, Phosphoniumtriflaten, Sulfoniumacetaten, Sulfoniumsulfonaten, Sulfoniumchloriden, Sulfoniumsulfaten, Sulfoniumphosphaten, Sulfoniumthiocyanaten, Sulfoniumdicyanamiden, Sulfoniumbenzoaten, Sulfoniumtriflaten, Ammoniumacetaten, Ammoniumsulfonaten, Ammoniumchloriden, Ammoniumsulfaten, Ammoniumphosphaten, Ammoniumthiocyanaten, Ammoniumdicyanamiden, Ammoniumbenzoaten, Ammoniumtriflaten und Gemischen davon.

5. Stimulationselektrode nach einem der Ansprüche 1 bis 4, wobei die ionische Flüssigkeit aus der Gruppe ausgewählt ist, bestehend aus 1-Ethyl-3-methylimidazoliumacetat, 1-Ethyl-3-methylimidazoliummethansulfonat, 1-Ethyl-3-methylimidazoliumtrifluormethansulfonat, 1-Ethyl-3-methylimidazoliumchlorid, 1-Ethyl-3-methylimidazoliumethylsulfat, 1-Ethyl-3-methylimidazoliumdiethylphosphat, 1-Ethyl-3-methylimidazoliumthiocyanat, 1-Ethyl-3-methylimidazoliumdicyanamid, 1-Ethyl-3-methylimidazoliumbenzoat, Cholintrifluormethansulfonat, Cholinsaccharinat, Cholinacesulfamate, Cholin-N-cyclohexylsulfamat, Tris(2-hydroxyethyl)methylammoniummethylsulfat, 1-Ethyl-3-methylimidazoliumtetrafluorborat, 1-Allyl-3-ethylimidazolium-bis(trifluormethylsulfonyl)imid, Cholinacetat, Cholintrifluormethansulfonat und Gemischen davon, und stärker bevorzugt aus der Gruppe ausgewählt ist, bestehend aus 1-Ethyl-3-methylimidazoliumbenzoat, 1-Ethyl-3-methylimidazoliumtetrafluoroborat, 1-Ethyl-3-methylimidazoliummethansulfonat, 1-Ethyl-3-methylimidazoliumchlorid, 1-Ethyl-3-methylimidazoliumtrifluormethansulfonat, Cholintrifluormethansulfonat, 1-Ethyl-3-methylimidazoliumacetat, Cholinacetat, 1-Ethyl-3-methylimidazoliumdiethylphosphat, 1-Allyl-3-methylimidazolium-bis(trifluormethylsulfonyl)imid, 1-Ethyl-3-methylimidazoliumethylsulfat, 1-Ethyl-3-methylimidazoliumthiocyanat, 1-Ethyl-3-methylimidazoliumdicyanamid, Cholinsaccharinat, Cholinacesulfamat und Gemischen davon.

6. Stimulationselektrode nach einem der Ansprüche 1 bis 5, wobei die ionische Flüssigkeit von 0,1 bis 50 Gew.-% des Gesamtgewichts der Zusammensetzung, vorzugsweise von 1 bis 35 Gew.-% und stärker bevorzugt von 4 bis 25 Gew.-% vorhanden ist.

7. Stimulationselektrode nach einem der Ansprüche 1 bis 6, wobei der Gehalt des (Meth)acrylatmonomers, umfassend die OH-Gruppe, in dem (Meth)acrylatharz mindestens 15 Gew.-% des Gesamtgewichts des (Meth)acrylatharzes, vorzugsweise mindestens 20 Gew.-%, stärker bevorzugt mindestens 25 Gew.-% und am stärksten bevorzugt mindestens 30 Gew.-%, aber nicht mehr als 65 Gew.-%, vorzugsweise nicht mehr als 60 Gew.-%, stärker bevorzugt nicht mehr als 55 Gew.-% und am stärksten bevorzugt nicht mehr als 50 Gew.-% beträgt.

8. Stimulationselektrode nach einem der Ansprüche 1 bis 7, ferner umfassend einen ionischen Leitfähigkeitspromotor, der vorzugsweise aus der Gruppe ausgewählt ist, bestehend aus Cholinchlorid, Cholinbitartrat, Cholindihydrogencitrat, Cholinphosphat, Cholingluconat, Cholinfumarat, Cholincarbonat, Cholinpyrophosphat und Gemischen davon.

9. Stimulationselektrode nach Anspruch 8, wobei der ionische leitfähige Promoter von 0,1 bis 35 Gew.-% des Gesamtgewichts der Zusammensetzung, vorzugsweise von 0,5 bis 25 Gew.-% und stärker bevorzugt von 1 bis 15 Gew.-% vorhanden ist.

10. Stimulationselektrode nach einem der Ansprüche 1 bis 9, ferner umfassend elektrisch leitfähige Partikel, die vorzugsweise aus der Gruppe ausgewählt sind, bestehend aus Metallpartikeln und Metallnanopartikeln, metallhaltigen Partikeln und Nanopartikeln, Graphitpartikeln und Nanopartikeln, Kohlenstoffpartikeln und Nanopartikeln, Kohlenstoffnanodrähten, leitfähigen Polymerpartikeln und Nanopartikeln und Gemischen davon, stärker bevorzugt aus der Gruppe ausgewählt, bestehend aus silberhaltigen Partikeln, Silberpartikeln, Kupferpartikeln, kupferhaltigen Partikeln, Silbernanodrähten, Kupfernanodrähten, Graphitpartikeln, Kohlenstoffpartikeln und Gemischen davon, und noch stärker bevorzugt aus Graphitpartikeln, Kohlenstoffpartikeln und Gemischen davon ausgewählt sind.

11. Stimulationselektrode nach Anspruch 10, wobei die elektrisch leitfähigen Partikel von 0,01 bis 30 Gew.-% des Gesamtgewichts der Zusammensetzung, vorzugsweise von 0,1 bis 25 Gew.-% und stärker bevorzugt von 1 bis 20 Gew.-% vorhanden sind.

12. Stimulationselektrode nach einem der Ansprüche 1 bis 11, ferner umfassend ein Polyetherpolyol, das vorzugsweise aus Polyethylenglykol, Polypropylenglykol, Polytetramethylenglykol und stärker bevorzugt Polyethylenglykol ausgewählt ist, das ein gewichtsmittleres Molekulargewicht von 300 bis 1000, stärker bevorzugt von 350 bis 750 und noch stärker bevorzugt von 380 bis 420 g/mol aufweist, wobei das Molekulargewicht durch eine Gelpermeationschromatographie gemäß DIN 55672-1:2007-08 mit THF als der Eluent gemessen wird.

13. Stimulationselektrode nach Anspruch 12, wobei das Polyetherpolyol von 0,01 bis 50 Gew.-% des Gesamtgewichts der Zusammensetzung, bevorzugt von 0,1 bis 25 Gew.-% und stärker bevorzugt von 1 bis 20 Gew.-% vorhanden ist.

14. Stimulationselektrode nach einem der Ansprüche 1 bis 13, wobei der Klebstoff einen Impedanzwert von unter 1.000.000 Ohm bei 1.000 Hz, aber mehr als 1.000 Ohm bei 1.000 Hz, vorzugsweise unter 100.000 Ohm bei 1.000 Hz, aber mehr als 5.000 Ohm bei 1.000 Hz und stärker bevorzugt unter 40.000 Ohm bei 1.000 Hz, aber mehr als 8.000 Ohm bei 1.000 Hz aufweist, wobei die Impedanz durch Verbinden zweier Elektroden gemessen wird, die jeweils mit 25 µm eines ionisch leitfähigen, druckempfindlichen Klebstoffs beschichtet sind, die eine Kontaktfläche von 0,25 cm² aufweisen.

15. Stimulationselektrode nach einem der Ansprüche 1 bis 14, wobei die Elektrode ferner b) eine leitfähige Schicht, die in Kontakt mit der ionisch leitfähigen, druckempfindlichen Klebeschicht steht; c) optional ein Substrat, das mit der leitfähigen Schicht in Kontakt steht; d) optional eine Trennfolie, die mit der leitfähigen, druckempfindlichen Klebeschicht in Kontakt steht; und e) optional einen Kontakt zwischen der Elektrode und der Stimulationsvorrichtung, umfasst.

## Revendications

1. Électrode de stimulation comprenant une composition d'adhésif sensible à la pression conducteur d'ions, comprenant
a) une résine (méth)acrylate comprenant au moins 10 % d'un monomère (méth)acrylate comprenant un groupe OH, en poids du poids total de la résine (méth)acrylate ; et
b) un liquide ionique,
dans laquelle l'électrode de stimulation comprend moins de 1 %, en poids du poids total de l'adhésif, d'un solvant, est exempte d'eau et ne contient pas d'hydrogel.

2. Électrode de stimulation selon la revendication 1, dans laquelle ladite résine (méth)acrylate est formée à partir des monomères choisis dans le groupe constitué d'acrylate d'hydroxyéthyle, acrylate d'hydroxypropyle, acrylate d'hydroxybutyle, méthacrylate de méthyle, acrylate de butyle, acrylate d'éthylhexyle, acide acrylique, (méth)acrylate d'alkyle en C1 à C18, (méth)acrylamide, acétate de vinyle, N-vinyl caprolactame, acrylonitrile, éther de vinyle, (méth)acrylate de benzyle, (méth)acrylate de cyclohexyle, (méth)acrylate de glycidyle et mélanges de ceux-ci, de préférence formée à partir des monomères choisis dans le groupe constitué d'acrylate d'hydroxyéthyle, méthacrylate de méthyle, acrylate de butyle, acrylate d'éthylhexyle et mélanges de ceux-ci, et plus préférablement ladite résine (méth)acrylate est formée à partir d'acrylate d'hydroxyéthyle, (méth)acrylate de méthyle, acrylate de butyle et acrylate d'éthylhexyle.

3. Électrode de stimulation selon la revendication 1 ou 2, dans laquelle ladite résine (méth)acrylate est présente à raison de 10 à 99 % en poids du poids total de la composition, de préférence de 25 à 98 % et plus préférablement de 50 à 95 %.

4. Électrode de stimulation selon l'une quelconque des revendications 1 à 3, dans laquelle le liquide ionique est choisi dans le groupe constitué d'acétates d'imidazolium, sulfonates d'imidazolium, chlorures d'imidazolium, sulfates d'imidazolium, phosphates d'imidazolium, thiocyanates d'imidazolium, dicyanamides d'imidazolium, benzoates d'imidazolium, triflates d'imidazolium, triflates de choline, saccharinate de choline, sulfamates de choline, acétates de pyridinium, sulfonates de pyridinium, chlorures de pyridinium, sulfates de pyridinium, phosphates de pyridinium, thiocyanates de pyridinium, dicyanamides de pyridinium, benzoates de pyridinium, triflates de pyridinium, acétates de pyrrolidinium, sulfonates de pyrrolidinium, chlorures de pyrrolidinium, sulfates de pyrrolidinium, phosphates de pyrrolidinium, thiocyanates de pyrrolidinium, dicyanamides de pyrrolidinium, benzoates de pyrrolidinium, triflates de pyrrolidinium, acétates de phosphonium, sulfonates de phosphonium, chlorures de phosphonium, sulfates de phosphonium, phosphates de phosphonium, thiocyanates de phosphonium, dicyanamides de phosphonium, benzoates de phosphonium, triflates de phosphonium, acétates de sulfonium, sulfonates de sulfonium, chlorures de sulfonium, sulfates de sulfonium, phosphates de sulfonium, thiocyanates de sulfonium, dicyanamides de sulfonium, benzoates de sulfonium, triflates de sulfonium, acétates d'ammonium, sulfonates d'ammonium, chlorures d'ammonium, sulfates d'ammonium, phosphates d'ammonium, thiocyanates d'ammonium, dicyanamides d'ammonium, benzoates d'ammonium, triflates d'ammonium et mélanges de ceux-ci.

5. Électrode de stimulation selon l'une quelconque des revendications 1 à 4, dans laquelle ledit liquide ionique est choisi dans le groupe constitué d'acétate de 1-éthyl-3-méthylimidazolium, méthanesulfonate de 1-éthyl-3-méthylimidazolium, trifluorométhanesulfonate de 1-éthyl-3-méthylimidazolium, chlorure de 1-éthyl-3-méthylimidazolium, éthylsulfate de 1-éthyl-3-méthylimidazolium, diéthylphosphate de 1-éthyl-3-méthylimidazolium, thiocyanate de 1-éthyl-3-méthylimidazolium, dicyanamide de 1-éthyl-3-méthylimidazolium, benzoate de 1-éthyl-3-méthylimidazolium, trifluorométhanesulfonate de choline, saccharinate de choline, acésulfamate de choline, N-cyclohexylsulfamate de choline, méthylsulfate de tris(2-hydroxyéthyl)méthylammonium, tétrafluoroborate de 1-éthyl-3-méthylimidazolium, bis(trifluorométhylsulfonyl)imide de 1-allyl-3-éthylimidazolium, acétate de choline et mélanges de ceux-ci, et plus préférablement choisi dans le groupe constitué de benzoate de 1-éthyl-3-méthylimidazolium, tétrafluoroborate de 1-éthyl-3-méthylimidazolium, méthanesulfonate de 1-éthyl-3-méthylimidazolium, chlorure de 1-éthyl-3-méthylimidazolium, trifluorométhanesulfonate de 1-éthyl-3-méthylimidazolium, trifluorométhanesulfonate de choline, acétate de 1-éthyl-3-méthylimidazolium, acétate de choline, diéthylphosphate de 1-éthyl-3-méthylimidazolium, bis(trifluorométhylsulfonyl)imide de 1-allyl-3-méthylimidazolium, éthylsulfate de 1-éthyl-3-méthylimidazolium, thiocyanate de 1-éthyl-3-méthylimidazolium, dicyanamide de 1-éthyl-3-méthylimidazolium, saccharinate de choline, acésulfamate de choline, et mélange de ceux-ci.

6. Électrode de stimulation selon l'une quelconque des revendications 1 à 5, dans laquelle ledit liquide ionique est présent à raison de 0,1 à 50 % en poids du poids total de la composition, de préférence de 1 à 35 % et plus préférablement de 4 à 25 %.

7. Électrode de stimulation selon l'une quelconque des revendications 1 à 6, dans laquelle la teneur dudit monomère (méth)acrylate comprenant un groupe OH dans ladite résine (méth)acrylate est d'au moins 15 % en poids du poids total de la résine (méth)acrylate, de préférence au moins 20 %, plus préférablement au moins 25 %, et le plus préférablement au moins 30 %, mais pas plus de 65 %, de préférence pas plus de 60 %, plus préférablement pas plus de 55 %, et le plus préférablement pas plus de 50 %.

8. Électrode de stimulation selon l'une quelconque des revendications 1 à 7, comprenant en outre un promoteur de conductivité ionique, choisi de préférence dans le groupe constitué de chlorure de choline, bitartrate de choline, dihydrogénocitrate de choline, phosphate de choline, gluconate de choline, fumarate de choline, carbonate de choline, pyrophosphate de choline et mélange de ceux-ci.

9. Électrode de stimulation selon la revendication 8, dans laquelle ledit promoteur de conductivité ionique est présent à raison de 0,1 à 35 % en poids du poids total de la composition, de préférence de 0,5 à 25 %, et plus préférablement de 1 à 15 %.

10. Électrode de stimulation selon l'une quelconque des revendications 1 à 9, comprenant en outre des particules électroconductrices, choisies de préférence dans le groupe constitué de particules de métal et nanoparticules de métal, particules et nanoparticules contenant du métal, particules et nanoparticules de graphite, particules et nanoparticules de carbone, nanofils de carbone, particules et nanoparticules de polymère conducteur, et mélanges de ceux-ci, plus préférablement choisies dans le groupe constitué de particules contenant de l'argent, particules d'argent, particules de cuivre, particules contenant du cuivre, nanofils d'argent, nanofils de cuivre, particules de graphite, particules de carbone et mélanges de ceux-ci, et même plus préférablement choisies parmi particules de graphite, particules de carbone et mélanges de celles-ci.

11. Électrode de stimulation selon la revendication 10, dans laquelle lesdites particules conductrices d'électricité sont présentes à raison de 0,01 à 30 % en poids du poids total de la composition, de préférence de 0,1 à 25 %, et plus préférablement de 1 à 20 %.

12. Électrode de stimulation selon l'une quelconque des revendications 1 à 11, comprenant en outre un polyol de polyéther, choisi de préférence parmi polyéthylène glycol, polypropylène glycol, polytétraméthylène glycol, et plus préférablement polyéthylène glycol ayant une masse moléculaire moyenne en poids allant de 300 à 1 000, plus préférablement de 350 à 750 et même plus préférablement de 380 à 420 g/mol, dans laquelle la masse moléculaire est mesurée par chromatographie par perméation sur gel selon la norme DIN 55672-1:2007-08 avec du THF comme éluant.

13. Électrode de stimulation selon la revendication 12, dans laquelle ledit polyol de polyéther est présent à raison de 0,01 à 50 % en poids du poids total de la composition, de préférence de 0,1 à 25 % et plus préférablement de 1 à 20 %.

14. Électrode de stimulation selon l'une quelconque des revendications 1 à 13, dans laquelle ledit adhésif a une valeur d'impédance inférieure à 1 000 000 Ohm à 1 000 Hz, mais supérieure à 1 000 Ohm à 1 000 Hz, de préférence inférieure à 100 000 Ohm à 1 000 Hz, mais supérieure à 5 000 Ohm à 1 000 Hz et plus préférablement inférieure à 40 000 Ohm à 1 000 Hz, mais supérieure à 8 000 Ohm à 1 000 Hz, dans laquelle ladite impédance est mesurée en connectant deux électrodes recouvertes chacune de 25 µm d'un adhésif sensible à la pression conducteur d'ions ayant une surface de contact de 0,25 cm².

15. Électrode de stimulation selon l'une quelconque des revendications 1 à 14, dans laquelle ladite électrode comprend en outre b) une couche conductrice, qui est en contact avec ladite couche d'adhésif sensible à la pression conducteur d'ions ; c) facultativement un substrat, qui est en contact avec la couche conductrice ; d) facultativement un revêtement anti-adhésif, qui est en contact avec la couche d'adhésif conducteur sensible à la pression ; et e) facultativement un contact entre ladite électrode et ledit dispositif de stimulation.
